# EUROPEAN PATENT SPECIFICATION

(11) **EP 2 385 808 B1**
(45) Date of publication and mention of the grant of the patent: **13.03.2013**
(21) Application number: 11701185.8
(22) Date of filing: 19.01.2011
(51) Int. Cl.: A61F 2/18, H04R 25/00

(54) **INCUS REPLACEMENT PARTIAL OSSICULAR REPLACEMENT PROSTHESIS**
GEHÖRKNÖCHELTEILERSATZPROTHESE ZUM ERSETZEN DES AMBOSS
PROTHÈSES DE REMPLACEMENT OSSICULAIRE PARTIEL POUR REMPLACEMENT D'ENCLUME

(30) Priority: 21.01.2010 US 296928 P; 20.07.2010 US 365824 P
(43) Date of publication of application: 16.11.2011
(60) Divisional application: 13151327.7
(73) Proprietor: Med-El Elektromedizinische Geraete GmbH, 6020 Innsbruck (AT)
(72) Inventor: BALL, Geoffrey R., 6094 Axams (AT); SANTEK, Michael, 6020 Innsbruck (AT); LENARZ, Thomas, 30559 Hannover (DE)
(74) Representative: Downing, Michael Philip
(86) International application number: PCT/US2011/021637
(87) International publication number: WO 2011/090974

(56) References cited:
- EP-A1- 1 759 662
- EP-A1- 2 135 584
- DE-U1-202005 015 533
- US-A- 3 899 822
- US-A1- 2007 249 890
- US-A1- 2007 255 405

## Description

### FIELD OF THE INVENTION

The present invention relates to medical implants, and more specifically to a novel ossicular prosthesis arrangement.

### BACKGROUND ART

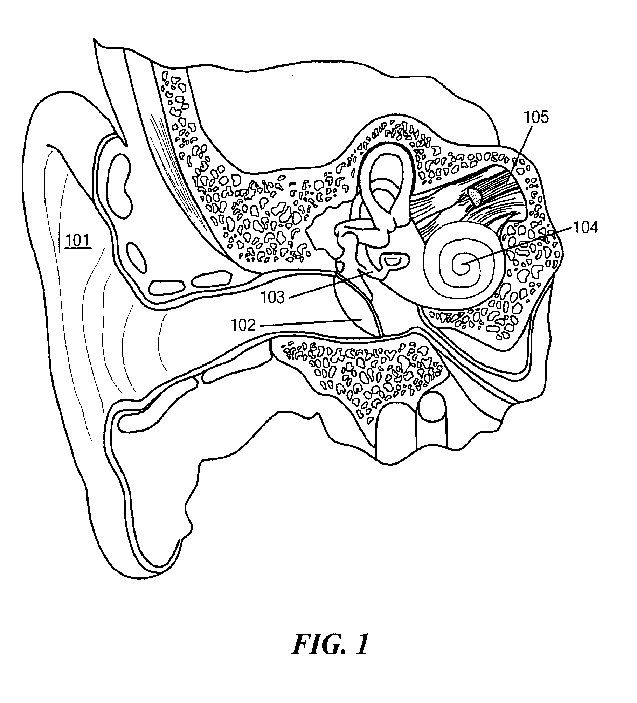

A normal ear transmits sounds as shown in Figure 1 through the outer ear 101 to the tympanic membrane (eardrum) 102, which moves the ossicles of the middle ear 103 (malleus, incus, and stapes) that vibrate the oval window and round window openings of the cochlea 104. The cochlea 104 is a long narrow duct wound spirally about its axis for approximately two and a half turns. It includes an upper channel known as the scala vestibuli and a lower channel known as the scala tympani, which are connected by the cochlear duct. The cochlea 104 forms an upright spiraling cone with a center called the modiolar where the spiral ganglion cells of the acoustic nerve 105 reside. In response to received sounds transmitted by the middle ear 103, the fluid-filled cochlea 104 functions as a transducer to generate electric pulses which are transmitted to the cochlear nerve 105, and ultimately to the brain.

Hearing is impaired when there are problems in the ability to transduce external sounds into meaningful action potentials along the neural substrate of the cochlea 104. To improve impaired hearing, auditory prostheses have been developed. For example, when the impairment is related to operation of the middle ear 103, a conventional hearing aid may be used to provide acoustic-mechanical stimulation to the auditory system in the form of amplified sound.

Middle ear implants also have been developed that employ electromagnetic transducers to mechanically stimulate the structures of the middle ear **103.** A coil winding is held stationary by attachment to a non-vibrating structure within the middle ear **103** and a microphone signal current is delivered to the coil winding to generate an electromagnetic field. A magnet is attached to an ossicle within the middle ear **103** so that the magnetic field of the magnet interacts with the magnetic field of the coil. The magnet vibrates in response to the interaction of the magnetic fields, causing vibration of the bones of the middle ear **103.** *See* U.S. Patent 6,190,305.

Middle ear implants using electromagnetic transducers can present some problems. Many are installed using complex surgical procedures which present the usual risks associated with major surgery and which also require disarticulating (disconnecting) one or more of the bones of the middle ear **103.** Disarticulation deprives the patient of any residual hearing he or she may have had prior to surgery, placing the patient in a worsened position if the implanted device is later found to be ineffective in improving the patient's hearing. US3899822 discloses an ossicular prosthesis including a wire shaped like a shepherd's crook for encircling the incus and which may be crimped thereto.

### SUMMARY OF THE INVENTION

Embodiments of the present invention are directed to an ossicular prosthesis which includes an elongated prosthesis member having a proximal end and a distal end. A cochlea striker mass is at the distal end of the prosthesis member and includes an outer striking surface for coupling vibration of the striker mass to an outer cochlea surface of a recipient patient. A locking clamp is at the proximal end of the prosthesis member and includes a clamp strap having a fixed end and a free end, and a locking head at the fixed end of the clamp strap which has a strap opening for insertion of the free end of the clamp strap. The clamp strap passes around an ossicle of the middle ear (e.g., a disarticulated incus) in a closed loop and is fixedly engaged by the locking head such that acoustic vibration of the ossicle is coupled by the prosthesis member to the cochlea surface.

The cochlea surface may include the round window membrane and/or the oval window membrane of the cochlea. The locking clamp may be a cable tie-type clamp. The prosthesis member may be made of titanium. The clamp strap may be made of plastic or a polymer material.

Embodiments of the present invention also include an ossicular connector having an elongated connector member. At least one end of the connector member includes a locking clamp having a clamp strap with a fixed end and a free end. A locking head is at the fixed end of the clamp strap and has a strap opening for insertion of the free end of the clamp strap. The clamp strap passes around an ossicle of the middle ear (e.g., a disarticulated incus) and an implanted acoustic signal transducer in a closed loop and is fixedly engaged by the locking head for attaching the signal transducer to the ossicle such that acoustic vibration of the signal transducer is coupled to the ossicle.

The connector member may have a locking clamp at each end for attaching the signal transducer to the ossicle. The locking clamp may be a cable tie-type clamp. The prosthesis member may be made of titanium. The clamp strap may be made of plastic or a polymer material.

### BRIEF DESCRIPTION OF THE DRAWINGS

Figure 1 shows various anatomical structures of a normal human ear.

Figure 2A shows one embodiment of an ossicular prosthesis in an open position.

Figure 2B shows another embodiment of an ossicular prosthesis in a closed position.

Figure 3A shows another embodiment of an ossicular prosthesis in a closed position.

Figure 3B and 3C shows the ossicular prosthesis of Fig. 3A in situ in a recipient patient.

Figure 4A shows an elevated perspective view of another embodiment of an ossicular connector for coupling an acoustic signal transducer to an ossicle in the middle ear.

Figure 4B shows the ossicular prosthesis of Fig. 4A in situ in a recipient patient.

Figure 5 A-C shows perspective views of various ossicular connectors.

Figure 6A shows a single foldable plane structure for forming a middle ear prosthesis.

Figure 6B shows the structure of Fig. 6A as folded into an open middle ear prosthesis ready to be surgically attached.

### DETAILED DESCRIPTION OF SPECIFIC EMBODIMENTS

Various embodiments of the present invention are directed to an ossicular prosthesis specifically referred to as a partial ossicular replacement prosthesis (PORP). The PORP uses a locking clamp having a clamp strap to connect a prosthesis member to an ossicle so as to couple vibration from the ossicle to the outer cochlea surface of a recipient patient for otologic reconstruction thereby providing sound sensation to the patient.

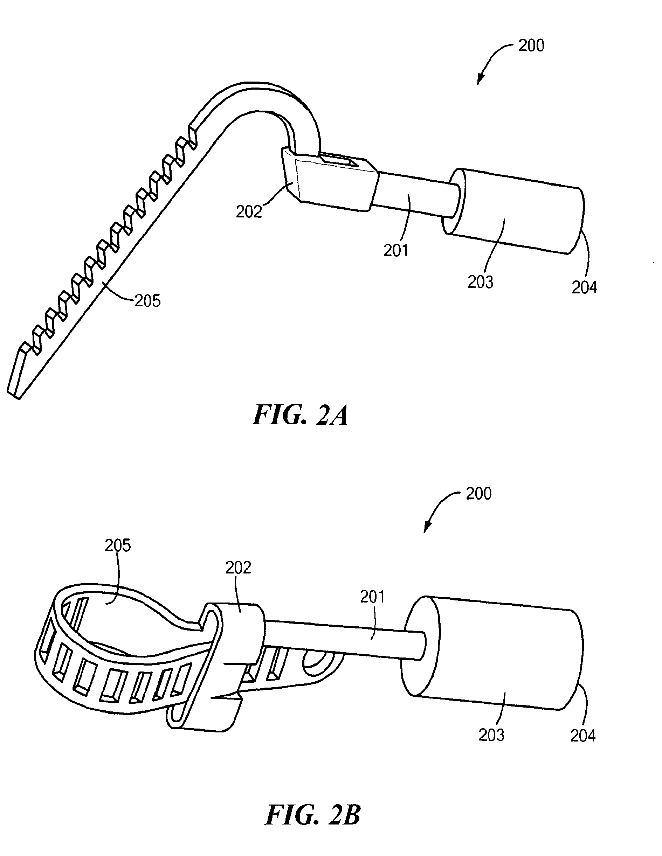

Figure 2 A-B shows two examples of a PORP ossicular prosthesis **200** which includes an elongated titanium prosthesis member **201** having a proximal end and a distal end. A cochlea striker mass **203** is at the distal end of the prosthesis member **201** and includes an outer striking surface **204** for coupling vibration of the striker mass **203** to an outer cochlea surface of a recipient patient. A locking clamp is at the proximal end of the prosthesis member **201** and includes a clamp strap **205** having a fixed end and a free end, and a locking head **202** at the fixed end of the clamp strap **205.** The locking head **202** has a strap opening for insertion of the free end of the clamp strap **205.** Thus, the locking clamp is basically a cable-tie or zip-lock type clamp which because the size of loop is adjustable is a one size fits all device.

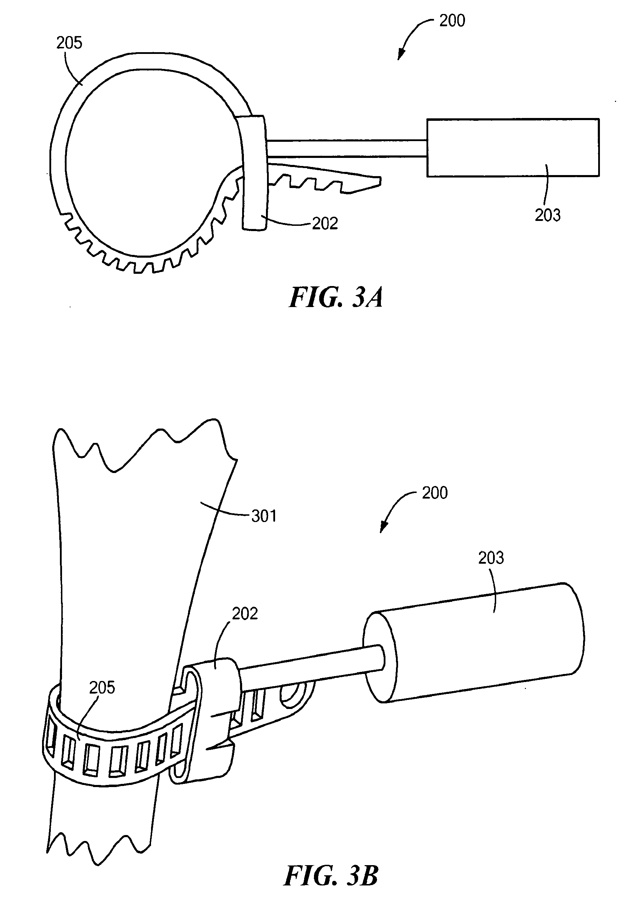

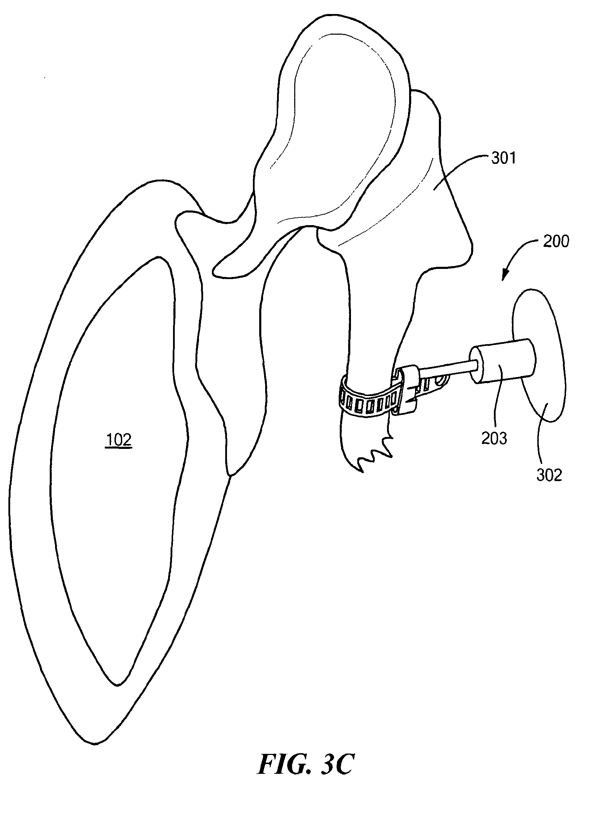

Fig. 3A shows a top view of a PROP ossicular prosthesis **200** as described above. Fig. 3B shows in close detail how the clamp strap **205** of the ossicular prosthesis **200** passes around an ossicle **301** of the middle ear (e.g., a disarticulated incus) in a closed loop and is fixedly engaged by the locking head **202** such that acoustic vibration of the ossicle **301** is coupled by the ossicular prosthesis **200** to a cochlea surface **302** of the patient such as the oval window membrane and/or the round window membrane. The clamp strap **205** is made of plastic or a polymer material so it can be tightened around the ossicle **301** and pulled snug with a desired amount of tension that can be dialed in by the surgeon. Fig. 3C shows the arrangement of Figs. 3A and 3B in situ in a recipient patient.

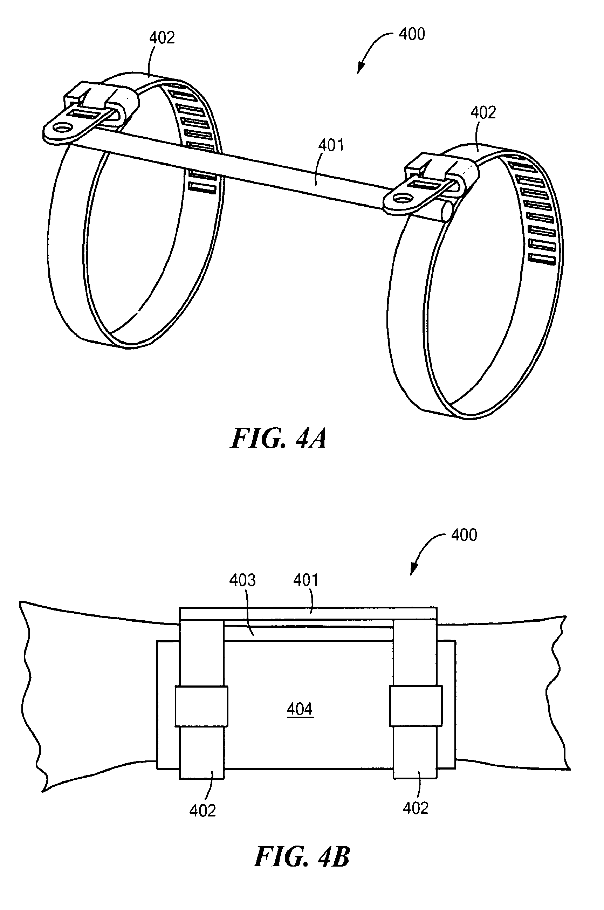

Fig. 4 A-B shows another embodiment of an ossicular connector **400** for coupling an acoustic signal transducer such as an implanted floating mass transducer (FMT) to an ossicle in the middle ear. The ossicular connector **400** includes an elongated titanium connector member **401,** at least one of which as a locking clamp **402** which includes a clamp strap and a locking head. In the embodiment shown in Fig. 4 A-B, there actually is a locking clamp **402** at each end of the connector member **401.** The clamp strap of the locking clamp **402** passes around an ossicle **403** of the middle ear (e.g., a disarticulated incus) and an implanted acoustic signal transducer **404** in a closed loop for attaching the signal transducer **404** to the ossicle **403** such that acoustic vibration of the signal transducer **404** is coupled to the ossicle **403,** and/or vice versa. The locking clamp **402** may specifically be a cable tie-type clamp. The clamp strap may be made of plastic or a polymer material.

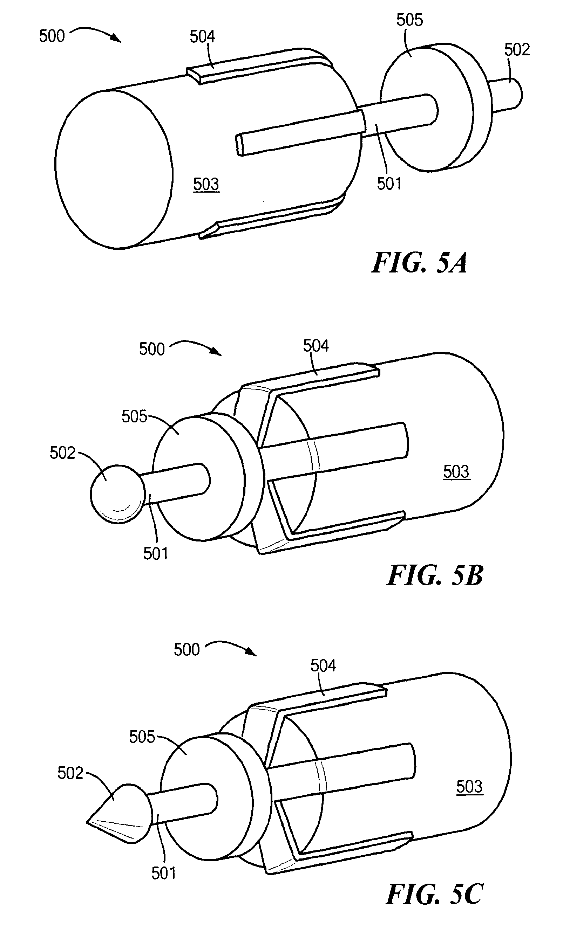

Fig. 5 A-C shows a prosthesis **500** for coupling an acoustic signal transducer **503** such as an FMT to the ossicular chain, for example, an ossicular bone or the outer surface of a patient cochlea such as the oval window or round window membranes. The middle ear prosthesis **500** includes an elongated titanium prosthesis member **501** with a proximal end and a distal end. A cochlea striker mass **502** is at the distal end of the prosthesis member **501** and has an outer striking surface for coupling vibration of the striker mass **502** to the ossicular chain of a recipient patient. For example, as shown in Fig. 5 A-C, the striking surface of the striker mass **502** may specifically be rod-shaped, conical, or spherical. The striker mass **502** may also include a resilient disc **505.** A transducer clamp is at the proximal end of the prosthesis member **501** and includes clamping fingers **504** for securely engaging the outer surface of an enclosed acoustic signal transducer **503** such that acoustic vibration of the signal transducer **503** is coupled by the prosthesis member **501** to the ossicular chain.

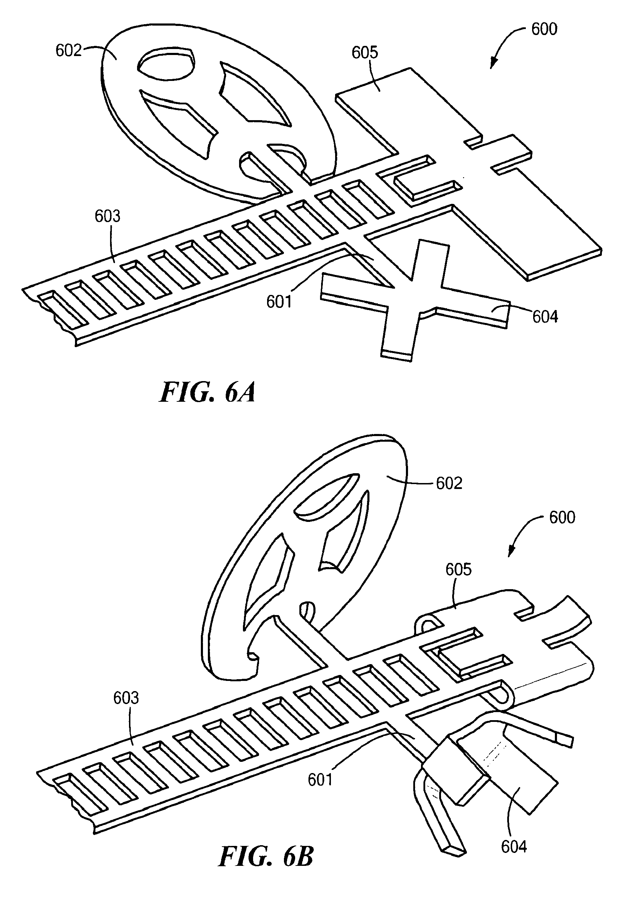

Figure 6 A-B shows another middle ear prosthesis **600** based on a structure formed from a single folded plane. Figure 6 A shows an example of a single planar sheet metal structure which is chemically etched as shown to have foldable sections **601-605.** Following the chemical etching of the metal plate into the desired shape, it may be bent with one or more manufacturing fixtures into the desired shapes as shown
in Fig. 6 B. In specific embodiments, some or all of a middle ear prosthesis **600** may be formed from such a single foldable plane structure: the prosthesis member **601** and the striker mass **602,** the prosthesis member **601** and the transducer clamp **604,** or the entire prosthesis **600** may be formed from a single foldable plane structure.

Embodiments of the present invention may be useful more generally in other surgeries for repair of structures where a clamp is called for, such as for limb repair other than in the middle ear. For example, a prosthesis member using a cable-tie type locking clamp to connect to a limb in some cases may avoid the need to use titanium screws. Similarly, a prosthetic strut may be implemented with a cable-tie type locking mechanism at either or both ends.

Although various exemplary embodiments of the invention have been disclosed, it should be apparent to those skilled in the art that various changes and modifications can be made which will achieve some of the advantages of the invention without departing from the true scope of the invention defined by the appended claims.

## Claims

1. An ossicular prosthesis (200) comprising:
an elongated prosthesis member (201) having a proximal end and a distal end;
a cochlea striker mass (203) at the distal end of the prosthesis member (201) including an outer striking surface (204) for coupling vibration of the striker mass (203) to an outer cochlea surface of a recipient patient; and **characterised by**
a locking clamp at the proximal end of the prosthesis member including:
1. a clamp strap (205) having a fixed end and a free end, and
n. a locking head (202) at the fixed end of the clamp strap (205) and having a strap opening for insertion of the free end of the clamp strap (205);
wherein the clamp strap (205) passes around an ossicle (301) of the middle ear in a closed loop and is fixedly engaged by the locking head (202) such that acoustic vibration of the
ossicle (301) is coupled by the prosthesis member (201) to the cochlea surface.

2. An ossicular prosthesis according to claim 1, wherein the cochlea surface includes the round window membrane of the cochlea.

3. An ossicular prosthesis according to claim 1, wherein the cochlea surface includes the oval window membrane of the cochlea.

4. An ossicular prosthesis according to claim 1, wherein the ossicle (301) is a disarticulated incus.

5. An ossicular prosthesis according to claim 1, wherein the locking clamp is a cable tie-type clamp.

6. An ossicular prosthesis according to claim 1, wherein the prosthesis member (201) is made of titanium.

7. An ossicular prosthesis according to claim 1, wherein the clamp strap (205) is made of plastic.

8. An ossicular prosthesis according to claim 1, wherein the clamp strap is made of a polymer material.

9. An ossicular connector comprising:
an elongated connector member (401), at least one end of which includes a locking clamp (402) having:
1. a clamp strap having a fixed end and a free end, and
n. a locking head at the fixed end of the clamp strap and having a strap opening for insertion of the free end of the clamp strap;
wherein the clamp strap passes around an ossicle (403) of the middle ear and an implanted acoustic signal transducer (404) in a closed loop and is fixedly engaged by the
locking head for attaching the signal transducer (404) to the ossicle (403) such that acoustic vibration of the signal transducer 404 is coupled to the ossicle 403.

10. An ossicular connector according to claim 9, wherein the connector member (401) has a locking clamp (402) at each end for attaching the signal transducer to the ossicle.

11. An ossicular connector according to claim 9, wherein the ossicle (403) is a disarticulated incus.

12. An ossicular connector according to claim 9, wherein the locking clamp (402) is a cable tie-type clamp.

13. An ossicular connector according to claim 9, wherein the connector member (401) is made of titanium.

14. An ossicular connector according to claim 9, wherein the clamp strap is made of plastic.

15. An ossicular connector according to claim 9, wherein the clamp strap is made of a polymer material.

## Patentansprüche

1. Gehörknöchelprothese (200), aufweisend:
ein längliches Prothesenelement (201) mit einem proximalen Ende und einem distalen Ende;
eine Cochlea-Schlagmasse (203) an dem distalen Ende des Prothesenelements (201), umfassend
eine äußere Aufschlagfläche (204) zum Einkoppeln einer Schwindung der Schlagmasse (203) in eine äußerte Cochleafläche eines Patienten, der der Empfänger ist; und **gekennzeichnet durch**
eine Sicherungsklammer an dem proximalen Ende des Prothesenelements, umfassend:
1. einen Klemmriemen (205) mit einem fixierten Ende und einem freien Ende,und
n. einen Sicherungskopf (202) an dem fixierten Ende des Klemmriemens (205) und mit einer Riemenöffnung zum Einführen des freien Endes des Klemmriemens (205);
wobei der Klemmriemen (205) in einem geschlossenen Bogen um ein Ossiculum (301) des Mittelohrs führt und der Sicherungskopf (202) fest in diesen eingreift, so dass eine akustische Schwingung des Ossiculums (301) **durch** das Prothesenelement (201) in die Cochleafläche gekoppelt wird.

2. Gehörknöchelprothese nach Anspruch 1, wobei die Cochleafläche die Rundfenstermembran der Cochlea umfasst.

3. Gehörknöchelprothese nach Anspruch 1, wobei die Cochleafläche die ovale Fenstermembran der Cochlea umfasst.

4. Gehörknöchelprothese nach Anspruch 1, wobei das Ossiculum (301) ein exartikulierter Amboß ist.

5. Gehörknöchelprothese nach Anspruch 1, wobei die Sicherungsklammer eine Klammer vom Kabelbindertyp ist.

6. Gehörknöchelprothese nach Anspruch 1, wobei das Prothesenelement (201) aus Titan gefertigt ist.

7. Gehörknöchelprothese nach Anspruch 1, wobei der Klemmriemen (205) aus Kunststoff gefertigt ist.

8. Gehörknöchelprothese nach Anspruch 1, wobei der Klemmriemen (205) aus einem Polymermaterial gefertigt ist.

9. Gehörknöchelverbindungsstück, aufweisend:
ein längliches Verbindungselement (401), von dem mindestens ein Ende eine Sicherungsklammer (402) umfasst, mit:
1. einem Klemmriemen mit einem fixierten Ende und einem freien Ende, und
n. einem Sicherungskopf an dem fixierten Ende des Klemmriemens und mit einer Riemenöffnung zum Einführen des freien Endes des Klemmriemens;
wobei der Klemmriemen in einem geschlossenen Bogen um das Ossiculum (403) des Mittelohrs und einen implantierten akustischen Signalgeber (404) geführt ist und der Sicherungskopf fest in diesen eingreift, um den Signalgeber (404) an dem Ossiculum (403) derart anzubringen, dass eine akustische Schwindung des Signalgebers (404) in das Ossiculum (403) eingekoppelt wird.

10. Gehörknöchelverbindungsstück nach Anspruch 9, wobei das Verbindungselement (401) an jedem Ende eine Sicherungsklammer (402) aufweiset, um den Signalgeber an dem Ossiculum anzubringen.

11. Gehörknöchelverbindungsstück nach Anspruch 9, wobei das Ossiculum (403) sein exartikulierter Amboss ist.

12. Gehörknöchelverbindungsstück nach Anspruch 9, wobei die Sicherungsklammer (402) eine Klammer vom Kabelbindertyp ist.

13. Gehörknöchetverbindungsstück nach Anspruch 9, wobei das Verbindungsstück (401) aus Titan gefertigt ist.

14. Gehörknöchelverbindungsstück nach Anspruch 9, wobei der Klemmriemen aus Kunststoff gefertigt ist.

15. Gehörknöchelverbindungsstück nach Anspruch 9, wobei der Klemmriemen aus einem Polymermaterial gefertigt ist.

## Revendications

1. Prothèse de remplacement ossiculaire (200) comprenant :
un élément de prosthèse (201) allongé ayant une extrémité proximale est une extrémité distale ;
une masse de percuteur (203) pour cochlée à l'extrémité distale de l'élément de prothèse (201) incluant une surface de percussion extérieure (204) pour couplage d'une vibration de la masse de percuter (203) à une surface extérieure de cochlée d'un patient receveur ; et **caractérisée par**
un élément de blocage à l'extrémité proximale de l'élément de prothèse incluant :
1. une bride de serrage (205) ayant une extrémité fixe et une extrémité libre, et
n. une tête de blocage (202) à l'extrémité fixe de la bride de serrage (205) et ayant une ouverture de bride pour insertion de l'extrémité libre de la bride de serrage (205) ;
dans laquelle la bride de serrage (205) passe autour d'un osselet (301) de l'oreille moyenne en une boucle fermée et est engagée de manière fixe par la tête de blocage (202) de telle manière qu'une vibration acoustique de l'osselet (301) est coupée par l'élément prosthèse (201) à la surface de la cochlée.

2. Prothèse de remplacement ossiculaire selon la revendication 1, dans laquelle la surface de la cochlée inclut la membrane de fenêtre ronde de la cochlée.

3. Prothèse de remplacement ossiculaire selon la revendication 1, dans laquelle la surface de la cochlée inclut la membrane de fenêtre ovale de la cochlée.

4. Prothèse de remplacement ossiculaire selon la revendication 1, dans laquelle l'osselet (301) est une enclume désarticulée.

5. Prothèse de remplacement ossiculaire selon la revendication 1, dans laquelle l'élément de blocage est un élément de type serre-câble.

6. Prothèse de remplacement ossiculaire selon la revendication 1, dans laquelle l'élément de prothèse (201) est constitué de titane.

7. Prothèse de remplacement ossiculaire selon la revendication 1, dans laquelle la bride de serrage (205) est constituée de plastique.

8. Prothèse de remplacement ossiculaire selon la revendication 1, dans laquelle la bride de serrage est constituée d'un matériau polymère.

9. Connecteur de remplacement ossiculaire comprenant :
un élément de connecteur (401) allongé, au moins une extrémité duquel inclut un élément de blocage (402) ayant :
1. une bride de serrage ayant une extrémité fixe et une extrémité libre, et
n. une tête de blocage à l'extrémité fixe de la bride de serrage et ayant une ouverture de bride pour insertion de l'extrémité libre de la bride de serrage ;
dans lequel la bride de serrage passe autour d'un osselet (403) de l'oreille moyenne et un transducteur de signaux acoustiques implanté (404) en une boucle fermée est engagé de façon fixe par la tête de blocage pour fixer le transducteur de signaux (404) à l'osselet (403) de telle manière qu'une vibration acoustique du transducteur de signaux (404) est couplée à l'osselet (403).

10. Connecteur de remplacement ossiculaire selon la revendication 9, dans lequel l'élément de connecteur (401) a un élément de blocage (402) à chaque extrémité pour fixer le transducteur de signaux à l'osselet.

11. Connecteur de remplacement ossiculaire selon la revendication 9, dans lequel l'osselet (403) est une enclume désarticulée.

12. Connecteur de remplacement ossiculaire selon la revendication 9, dans lequel l'élément de blocage (402) est un élément de type serre-câble.

13. Connecteur de remplacement ossiculaire selon la revendication 9, dans lequel l'élément de connecteur (401) est constitué de titane.

14. Connecteur de remplacement ossiculaire selon la revendication 9, dans lequel la bride de serrage est constituée de plastiqué.

15. Connecteur de remplacement ossiculaire selon la revendication 9, dans lequel la bride de serrage est constituée d'un matériau polymère.
